# EUROPEAN PATENT APPLICATION

(11) **EP 2 359 845 A1**
(43) Date of publication of application: **24.08.2011**
(21) Application number: 09830289.6
(22) Date of filing: 09.11.2009
(51) Int. Cl.: A61K 38/44, A61K 9/10, A61K 9/14, A61K 9/72, A61K 47/26, A61K 47/48, A61P 11/00

(54) **INHALANT COMPRISING MODIFIED SUPEROXIDE DISMUTASE**

(30) Priority: 03.12.2008 JP 2008308331
(71) Applicant: LTT Bio-Pharma Co., Ltd., Minato-ku Tokyo 105-0022 (JP)
(72) Inventor: MIZUSHIMA, Toru, Kumamoto-shi Kumamoto 862-0975 (JP)
(74) Representative: Albrecht, Thomas
(86) International application number: PCT/JP2009/069067
(87) International publication number: WO 2010/064522

(57) **Abstract**

An inhalant containing a lecithinized superoxide dismutase (hereinafter referred to as PC-SOD) which effectively exerts the effect of superoxide dismutase (SOD) as an active ingredient, particularly for treatment of idiopathic (acute) or chronic interstitial pneumonia, is provided. The inhalant includes a PC-SOD represented by the following general formula (I):

SOD'(Q-B)ₘ (I)

(wherein SOD' is a residue of the superoxide dismutase; Q is a chemical crosslinking; B is a residue without a hydrogen atom of a hydroxyl group of lysolecithin having the hydroxyl group at the 2-position of glycerol; and m is the average number of bonds of lysolecithin to one molecule of superoxide dismutase and is an integer of 1 or more).

## Description

### TECHNICAL FIELD

The present invention relates to an inhalant containing a superoxide dismutase, and particularly to an inhalant containing a lecithinized superoxide dismutase (hereinafter may be simply referred to as PC-SOD) as an active ingredient, particularly for treatment of lung diseases such as interstitial pneumonia.

### BACKGROUND ART

A superoxide dismutase (hereinafter may be simply referred to as SOD) is a bioactive protein which has been extracted from bovine blood as an anti-inflammatory protein by Huber et al., in 1965, and which has been found that it specifically eliminates superoxide anions (O₂⁻) as one of active oxygen species. In the living organism, active oxygen is mainly released by phagocytes such as neutrophils and macrophages for sterilization. However, there are typically various antioxidants such as SODs corresponding to excessive active oxygen, and they protect healthy cells against injury by the active oxygen.

However, when there is excessive active oxygen beyond the antioxidant ability of the antioxidants such as SODs, substances near the active oxygen, particularly cell membranes are attacked by the active oxygen to develop various disease conditions. In fact, since it has been proven that the active oxygen has potent tissue damage properties, it has been revealed that it becomes causative or precipitating factors of many disease conditions such as inflammation, allergy, tissue damage caused by ischemia reperfusion, and fibroid lung caused by anticancer drugs.

Under such circumstances, an SOD which specifically eliminates active oxygen has been found, and the possibility of clinical application has been widely investigated. The present inventor also has investigated clinical application of SOD earnestly. The inventors then have believed that it is important to maintain the blood level of SOD by reducing clearance from the kidney to enhance clinical effects of SOD, and to eliminate excessive active oxygen present on the cell membranes by enhancing affinity for the cell membranes. The inventor has investigated various modified SODs, and proposed a lecithinized superoxide dismutase (PC-SOD) (Patent Documents 1 and 9).

The PC-SOD is a lecithinized SOD obtained by preparing a Cu- and Zn-containing human superoxide dismutase (SOD) by gene recombination technology, and then performing chemical binding of an average of four molecules of lecithin derivative (phosphatidylcholine derivative: PC) to one molecule of SOD (dimmer). The PC-SOD has high affinity for cell membranes, and is approved to have high therapeutic effects on diseases involving active oxygen, such as ischemia-reperfusion injury and cardiomyopathy induced by anthracycline anticancer drugs which are etiological factors in the lesions. Carious agents containing a PC-SOD as an active ingredient, such as a therapeutic agent for acute heart failure (Patent Document 2), an antiviral agent (Patent Document 3), a therapeutic agent for lupus nephritis (Patent Document 4), an improving agent for cerebral vascular accident-related dysfunction (Patent Document 5), an anti-fibrosis agent (Patent Document 6), or a treatment agent for allergic diseases (Patent Document 7), and a therapeutic agent for burns (Patent Document 8) have been already proposed.

Pneumonia is an infectious disease, in which pathogens invade the alveolar area, grow, and induce biological reaction. Interstitial pneumonia is a disease, in which a main lesion (primarily including thickening, cell infiltration, and fibrosis) is alveolar septa, and inflammation and fibrosis are protracted. The interstitial pneumonia can be classified depending on whether or not their etiology is known. The interstitial pneumonia is an intractable disease, in which inflammation causes increase in cells, collagen, and the like, to thicken the alveolar wall, and reduce oxygen uptake, resulting in shortness of breath (dyspnea).
Although some cases of interstitial pneumonia are transient, in most cases, hardening in the lung progresses gradually and deteriorates irreversibly, to harden the lung, developing fibroid lung, in which breathing cannot be maintained.

In these interstitial pneumonias, idiopathic interstitial pneumonia (IIP) is a generic term of interstitial pneumonias of unknown etiology, rather than a single disease. IIP is generally a synonym for idiopathic pulmonary fibrosis (IPF) used in the US. In Japan, IIP is a disease name put forward by a team of Japanese researchers studying interstitial pneumonias in 1981. The symptoms of IIP mostly progress slowly, while rapid progress may be developed. In either case, the prognosis will be poor, often progressing to death.
In Japan, IIP is classified into idiopathic pneumatic fibrosis, nonspecific interstitial pneumonia, acute interstitial pneumonia, idiopathic cryptogenic organizing pneumonia, respiratory bronchiolitis-associated interstitial lung disease, desquamative interstitial pneumonia, lymphocytic interstitial pneumonia, and the like, as a clinicopathological disease entity.

The causes of idiopathic interstitial pneumonias are not known, and inflammation and immunity are suspected to be involved in fibrosis of the lung, in addition to various genetic backgrounds.
Recently, it has been discovered that interstitial pneumonia occurs as a side effect of antitumor drugs, and in particular, interstitial pneumonia becomes a problem as a serious side effect of gefitinib.

In either case, induction of cytotoxic effects or induction of allergy reactions according to onset of interstitial pneumonia involves active oxygen such as superoxide anions, and iron complexes. Thus, it is believed that elimination of active oxygen caused by SODs can suppress these inductions and as a result, interstitial pneumonia can be treated.
From this viewpoints, the inventors have investigated therapeutic effects for patients with interstitial pneumonia, additionally with idiopathic interstitial pneumonia, by using the previously proposed lecithinized superoxide dismutase (PC-SOD) having high affinity for cells. As a result, the inventors have confirmed that the PC-SOD exerts effective therapeutic effects.

However, the effects cannot be sufficient, and additional improvement has been requested.
Under such circumstances, the inventor has additionally investigated, and confirmed that intratracheal administration, in which a PC-SOD is directly administered to a lung tissue, or spray administration maintains a PC-SOD concentration in the lung tissue at a high level and the PC-SOD is extremely effective for interstitial pneumonia. The present invention has been completed.
Heretofore, there have not been specific approaches, in which a PC-SOD itself is directly intratracheally administered to a lung tissue to treat interstitial pneumonia, and particularly idiopathic interstitial pneumonia. In this context, the present invention is very unique.

Patent document 1: Japanese Patent Application Laid-Open No. Hei 9-117279
Patent document 2: Japanese Patent Application Laid-Open No. Hei 9-52843
Patent document 3: Japanese Patent Application Laid-Open No. Hei 9-59178
Patent document 4: Japanese Patent Application Laid-Open No. Hei 9-110717
Patent document 5: Japanese Patent Application Laid-Open No. Hei 10-338645
Patent document 6: Japanese Patent Application Laid-Open No. 2001-2585
Patent document 7: Japanese Patent Application Laid-Open No. 2001-151695
Patent document 8: Japanese Patent Application Laid-Open No. 2006-169128
Patent document 9: Japanese Patent Application Laid-Open No. 2001-64199

### DISCLOSURE OF INVENTION

### PROBLEMS TO BE SOLVED BY THE INVENTION

In view of the problems, it is an object of the present invention to provide an inhalant containing a PC-SOD as an active ingredient for treatment of interstitial pneumonia, and particularly idiopathic (acute) or chronic interstitial pneumonia.

### MEANS FOR SOLVING THE PROBLEMS

To solve the problem, one basic aspect of the present invention is an inhalant containing, as an active ingredient, a lecithinized superoxide dismutase represented by the following general formula (I):

SOD' (Q-B)ₘ (I)

(wherein SOD' is a residue of a superoxide dismutase; Q is a chemical crosslinking; B is a residue without a hydrogen atom of a hydroxyl group of lysolecithin having the hydroxyl group at the 2-position of glycerol; and m is the average number of bonds of lysolecithin to one molecule of superoxide dismutase and is an integer of 1 or more).

The present invention is preferably an inhalant, wherein Q in the lecithinized superoxide dismutase represented by the formula (I) used in the present invention is -C-(O)-(CH₂)ₙ-C(O)- (wherein n is an integer of 2 or more).

Further, the present invention is specifically an inhalant, wherein the SOD' is a residue of a human superoxide dismutase, and more specifically a residue of a modified superoxide dismutase, in which the amino acid at the 111-position of amino acid sequence of the human superoxide dismutase is converted into S-(2-hydroxyethylthio)cysteine.

Most specifically, the present invention is an inhalant, wherein the superoxide dismutase is a superoxide dismutase containing copper and zinc at the active center.

The present invention is an inhalant containing a lecithinized superoxide dismutase and a stabilizing agent thereof, wherein the stabilizing agent is a sugar component, and especially sucrose.

Most specifically, the present invention is an inhalant which has a form of a fine powder formulation, an aqueous solution, or a suspension formulation for inhalation, and is intratracheally administered.

The present invention is an inhalant for treatment or prevention of interstitial pneumonia, and especially an inhalant, in which interstitial pneumonia occurs as a side effect of antitumor drugs.

Interstitial pneumonia in the present invention includes idiopathic interstitial pneumonia, and includes, as a clinicopathological disease entity, diseases classified into idiopathic pneumatic fibrosis, nonspecific interstitial pneumonia, acute interstitial pneumonia, idiopathic cryptogenic organizing pneumonia, respiratory bronchiolitis-associated interstitial lung disease, desquamative interstitial pneumonia, lymphocytic interstitial pneumonia, and the like.

### EFFECT OF THE INVENTION

In the present invention, onset of interstitial pneumonia is caused by, for example, drug administration, induction of cytotoxic effects by metabolites thereof, or induction of allergy reactions. The inductions involve active oxygen such as superoxide anions, and iron complexes. Therefore, elimination of the active oxygen caused by SODs, or the like, can effectively suppress these inductions. As a result, effective treatment for interstitial pneumonia can be performed. The present invention can be used as an effective therapeutic agent for interstitial pneumonia which is one of side effects of an antitumor drug.
Under a circumstance where there have not been effective therapeutic agents for interstitial pneumonia heretofore, intratracheal administration of specific PC-SOD, particularly, enables administration of PC-SOD to a lung tissue at a high level. Therefore, there are advantages, in which the therapeutic effect is sufficiently high.

The PC-SOD used in the present invention has more excellent affinity for cell membranes than the conventional SOD and higher ability to eliminate superoxide anions locally in the lesion. In addition, when a sugar component, and particularly sucrose are contained together as the stabilizing agent, the PC-SOD itself becomes excellent in stability. Thus, the effect of an SOD having a short half-life is continuously exerted. In terms of effectively treating interstitial pneumonia, the PC-SOD is particularly excellent.

### BRIEF DESCRIPTION OF THE DRAWINGS

Fig. 1 is a view showing the whole number of cells in an alveolar lavage fluid in Example 2.
Fig. 2 is a view showing the results of alveolar macrophages in Example 2.
Fig. 3 is a view showing the results of lymphocytes in Example 2.
Fig. 4 is a view showing the results of neutrophils in Example 2.
Fig. 5 is a view showing the results of the amount of hydroxyproline in the lung tissues in Example 3.
Fig. 6 is a view showing the whole number of cells in an alveolar lavage fluid in Example 4.
Fig. 7 is a view showing the results of alveolar macrophages in Example 4.
Fig. 8 is a view showing the results of lymphocytes in Example 4.
Fig. 9 is a view showing the results of neutrophils in Example 4.

### BEST MODE FOR CARRYING OUT THE INVENTION

In the lecithinized superoxide dismutase (PC-SOD) used for the therapeutic agent for interstitial pneumonia provided by the present invention, "lecithin" is referred to as normal lecithin which means phosphatidylcholine, and "lysolecithin" is referred to as a compound, in which one molecule of fatty acid bonded at the 2-position of glycerol in lecithin is removed and a hydroxyl group is bound to the carbon atom at the 2-position.

In the present invention, the PC-SOD used as an active ingredient can be usually obtained by binding one or more lecithin derivatives, in which a chemical crosslinking agent is bound to the hydroxyl group at the 2-position of lysolecithin to the SOD. The PC-SOD can be represented by the following formula (I):

SOD'(Q-B)ₘ (I)

(wherein the SOD' is a residue of the superoxide dismutase; Q is a chemical crosslinking; B is a residue without a hydrogen atom of a hydroxyl group of lysolecithin having the hydroxyl group at the 2-position of glycerol; and m is the average number of bonds of lysolecithin to one molecule of superoxide dismutase and is an integer of 1 or more).

The SOD' used herein may be not particularly limited to an origin thereof as long as such an essential function of decomposing active oxygen (O₂⁻) in the living organism is exerted. SOD residues derived from various plants, animals, or microorganisms can be widely used. However, in view of application for medicines, it is preferable that antigenicity in the living organism be reduced as much as possible. Accordingly, as the SOD' for use, it is preferable to suitably select appropriate SOD residues depending on subjects to administer the therapeutic agent for interstitial pneumonia of the present invention.

For example, the SOD' is one attempting to be administered to actual patients with interstitial pneumonia as the subject, and therefore, in order to reduce antigenicity in the living organism as much as possible due to the administration, human-derived SOD residues are preferably used. Accordingly, as an SOD for treatment of interstitial pneumonia of the present invention, in view of antigenicity, the human-derived SOD is used better.

As the human-derived SOD, a human-derived Cu- and Zn-containing SOD (human-derived SOD containing copper and zinc at the active center; hereinafter may be abbreviated as human Cu- and Zn-containing SOD) is expressed in a large amount in cells, production technology based on a genetic engineering method has been already established, and therefore the human Cu- and Zn-containing SOD can be prepared in a large amount. Accordingly, the human Cu- and Zn-containing SOD is particularly preferably used.

This human Cu- and Zn-containing SOD includes: a natural human Cu- and Zn-containing SOD produced from human tissues or cultured cells; a human Cu- and Zn-containing SOD produced by the genetic engineering method; a recombinant human Cu- and Zn-containing SOD having substantially the same amino acid sequence as in the natural human Cu- and Zn-containing SOD; an SOD in which partial amino acids in amino acid sequences of these human Cu- and Zn-containing SODs are deleted, added, substituted, or chemically modified or changed; and the like, and any human Cu- and Zn-containing SOD may be used.

Among them, a human Cu- and Zn-containing SOD, in which an amino acid (cysteine: Cys) at the 111-position of amino acid sequence of natural human Cu- and Zn-containing SOD has been converted into S-(2-hydroxyethylthio)cysteine is preferable. Such a human Cu- and Zn-containing SOD is described in detail in Patent document 1 (Japanese Patent Laid-open Publication No. 9-117279), and can be obtained by the method described therein.
Accordingly, preparation of human Cu- and Zn-containing SOD described in detail in Patent Document 1 (Japanese Patent Application Laid-Open No. Hei 9-117279) is partially incorporated herein, and in the case of the PC-SOD used in the present invention, these human Cu- and Zn-containing SODs can be obtained as a material.

In the PC-SOD represented by the formula (I) used in the present invention, "a residue without a hydrogen atom of a hydroxyl group of lysolecithin having the hydroxyl group at the 2-position of glycerol" shown as B is specifically represented by the following formula (II):

-O-CH(CH₂OR) [CH₂OP(Q) (O⁻) (OCH₂CH₂N⁺(CH₃)₃)] (II)

(wherein R is a fatty acid residue (acyl group)).

The fatty acid residue (acyl group) shown as R is preferably a saturated or unsaturated fatty acid residue having a carbon number of 10 to 28, more preferably a myristoyl group, a palmitoyl group, a stearoyl group, an icosanoyl group, a docosanoyl group, and another saturated fatty acid residue having a carbon number of 14 to 22, and particularly preferably a palmitoyl group which is a saturated fatty acid residue having a carbon number of 16.

The chemical crosslinking shown as Q in the general formula (I) is not particularly limited as long as an SOD and lecithin can be crosslinked to be chemically (covalently) bonded with each other. Such a chemical crosslinking is particularly preferably a residue: - C(O)-(CH₂)ₙ-C(O)- (wherein n is an integer of 2 or more). This residue is a residue without hydroxyl groups at both the ends of a linear dicarboxylic acid represented by the formula: HO-C(O)-(CH₂)ₙ-C(O)-OH, an anhydride, an ester, or a halide thereof, or the like (provided that in the case of the anhydride, ester, and halide, a moiety corresponding to the hydroxy groups at both the ends).

When Q in the general formula (I) is the above-described linear dicarboxylic acid residue, one end of Q is bonded to oxygen of the hydroxyl group of lysolecithin residue represented by the formula (II) through an ester bond. In addition, the other end of Q which has been formed with an ester bond is directly bonded to an amino group of SOD through an amide bond, or the like.
In the residue of the above-described chemical crosslinking, n is an integer of 2 or more, and preferably an integer of 2 to 10.

In the formula (I), m represents the average number of bond of lysolecithin to one molecule of SOD. For this reason, m is an integer of 1 or more, preferably 1 to 12, and particularly preferably 4.

A method for producing a PC-SOD used in the present invention, that is, a method for binding a lecithin derivative with an SOD, and preferably with a human Cu- and Zn-containing SOD can be performed, for example, by using the method described in Patent document 1.

When the chemical structure of the preferable PC-SOD is schematically shown, the following PC-SOD is particularly preferable.

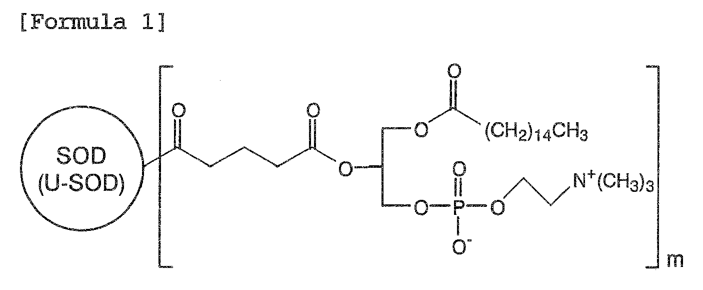

(wherein m is the number of bound lecithin derivatives).

In other words, the PC-SOD is obtained by covalent binding of an average of four molecules of a lecithin derivative to a free amino group of a human Cu- and Zn-containing SOD produced by gene recombination using E. coli as a host cell.

It is preferable that the PC-SOD used in the inhalant provided by the present invention, particularly for treatment of interstitial pneumonia be purified to such an extent that it is usable as a medicine and substantially contain no substances which are not permitted to be mixed as a medicine. For example, it is preferable that as the PC-SOD, a purified PC-SOD having a specific SOD activity of 2,500 U/mg (2.5 kU/mg) or more, and more preferably having a specific SOD activity of 3,000 U/mg (3.0 kU/mg) or more can be used.
In the present invention, 1 U (unit) represents the enzyme amount of PC-SOD which inhibits 50% of NBT (nitro blue tetrazolium) reduction rate as measured using NBT under a condition of pH 7.8 and 30°C in accordance with the method described in J. Biol. Chem., vol. 244, No. 22 6049-6055 (1969).

The inhalant provided by the present invention is an inhalant containing the PC-SOD thus prepared as an active ingredient. Such an inhalant means a pharmaceutical composition for delivery to the trachea, bronchus, lung, and the like, suitably a composition suitable for a nasal drop, or administration through the nose or lung, and particularly a composition suitable for administration through the lung.

The inhalant of the present invention can be produced in the form of powder, solution, or suspension using the above-described PC-SOD as an active ingredient.

When an inhalant is produced in a powder form, the PC-SOD as an active ingredient is pulverized as it is or with an additive such as an excipient, a lubricant, a binder, a disintegrator, a stabilizing agent, and a corrective, and therefore the inhalant can be produced.

Examples of excipients include organic excipients including saccharides such as lactose, sucrose, glucose, mannitol, and sorbitol; starch derivatives such as corn starch, potato starch, α-starch, dextrin and carboxymethyl starch; cellulose derivatives such as crystal cellulose, low-substituted hydroxypropylcellulose, hydroxypropyl methylcellulose, carboxymethyl cellulose, and carboxymethyl cellulose calcium; gum arabic, dextran, and pullulan; and inorganic excipients including silicic acid derivatives such as light anhydrous silicic acid, and synthetic aluminum silicate and magnesium aluminum silicate; phosphate salts such as calcium phosphate; carbonate salts such as calcium carbonate; and sulfate salts such as calcium sulfate.

Examples of lubricants include stearic acid, metal salts of stearic acid such as calcium stearate and magnesium stearate; talc; colloidal silica; waxes such as veegum or tspermaceti: boric acid; adipic acid; sodium sulfate; sulfate salts; glycol; fumaric acid; sodium benzoate; DL-leucine; fatty acid sodium salts; laurylsulfate salts such as sodium lauryl sulfate and magnesium lauryl sulfate; silicic acids such as silicic anhydride and silicic acid hydrates; and the above-described starch derivatives.

Examples of binders include polyvinyl pyrrolidone, macrogol, and the same compounds as in the excipient.

Examples of disintegrators include the same compounds as in the excipient, and chemically-modified starch and celluloses such as croscarmellose sodium, sodium starch glycolate, and crosslinked-polyvinyl pyrrolidone.

Examples of stabilizing agents include p-oxybenzoic acid esters such as methyl paraben and propyl paraben; alcohols such as chlorobutanol, benzyl alcohol and phenylethyl alcohol; benzalkonium chloride; phenols such as phenol and cresol; thimerosal; dehydroacetic acid; sorbic acids; and the like.
Examples of correctives include sweeteners, acidulants, flavors, and the like, which are usually used.

In the inhalant provided by the present invention, when the inhalant is produced as a solution or suspension, the inhalant can be produced by dissolving or suspending PC-SOD in water or a mixture of water and an auxiliary solvent, for example, an alcohol auxiliary solvent such as ethanol, propylene glycol, and polyethylene glycol. Such a solution or suspension can additionally contain antiseptics, solubilizers, buffers, isotonizing agents, absorption promoters, thickeners, and the like.

Examples of antiseptics include benzalkonium chloride, examples of solubilizers include polysorbate and a surfactant, and examples of isotonizing agents include sodium chloride. To the suspension, a suspending agent (for example, microcrystalline cellulose, and carboxymethyl cellulose sodium) may be further added.

The inhalant produced as described above is directly administered inside the nasal or mouth cavity or to the trachea, bronchi, lung, or the like in a nebulous form by using common means in the field of inhalant, for example, a dropper, a pipette, a cannula, or a sprayer such as an atomizer or a nebulizer. In the case of using a sprayer, the inhalant can be administered by spraying it as an aerosol in the form of pressure bag with an appropriate propellant (for example, gases of chlorofluorocarbons such as dichlorofluoromethane, trichlorofluoromethane, or dichlorotetrafluoroethane, carbon dioxide, or the like), or by using a nebulizer.

The inhalant of the present invention may be an inhalant containing a PC-SOD as an active ingredient and a stabilizing agent. Examples of the stabilizing agent include a sugar component. The sugar component is not particularly limited as long as it is a sugar component used pharmaceutically, and sucrose is preferable. Therefore, the most preferable inhalant provided by the present invention is a composition containing a PC-SOD and sucrose. As sucrose, sucrose purified to an extent usable as a medicine is preferably used, and in particular, sucrose processed by activated charcoal is preferably used. Such sucrose can be used together with a PC-SOD, whereby reduction in the activity of the PC-SOD due to long term storage can be prevented, and accordingly, sucrose can be used to prepare a composition for inhalation whose stability is high and property is particularly good even if it is lyophilized.

The mixing ratio of the PC-SOD to sucrose in the inhalant provided by the present invention, and particularly in the inhalant for treatment of interstitial pneumonia can be suitably determined depending on an administration amount, a form of the formulation, or the like, and is particularly limited.
However, the weight ratio of the PC-SOD to sucrose is preferably within a range of about 0.1/100 to 80/100, and more preferably about 0.4/100 to 60/100.

The amount of PC-SOD which is an active ingredient when the inhalant provided by the present invention is used to prepare a therapeutic agent for treatment of interstitial pneumonia and the administration amount of the formulation are varied depending on a method for preparing the formulation, a dosage form, a target disease degree, or age or body weight of a patient, but not particularly limited. For example, as a clinical amount, 0.5 to 100 mg (1,500 to 300,000 U) daily per adult can be exemplified. Further, the number of doses is not particularly limited, but the administration can be performed once or more daily.

### EXAMPLES

Hereinafter, the present invention will be described in detail by specific Examples. However, the present invention is not limited to these Examples.

### Example 1: Effect of PC-SOD for bleomycin-induced lung injury model mouse

To ICR mice (6- to 8-week old, body weight: 28 to 32 g), 5 mg/kg of bleomycin was intratracheally administered to produce lung injury.
In confirmation of production of lung injury, macrophages, lymphocytes, and neutrophils in an alveolar lavage fluid were measured to confirm the state of cell infiltration (inflammation state), and the amount of hydroxyproline (collagen) was measured to confirm the state of fibrosis.
To the lung injury model mice, PC-SODs with the respective concentrations were administered once daily by various administration routes including intravenous, intratracheal, and pulmonary inhalation administration.
The mice were sacrificed 6 hours after the final administration, a serum and a lung tissue were collected, and the concentration of the PC-SOD in the serum and lung tissue was measured by ELISA method.
The results are summarized in Table 1 described below.

**[Table 1]**

| PC-SOD administration amount | Administration Route | Concentration in serum (µg/ml) | Concentration in lung tissue (µg/g tissue) |
|---|---|---|---|
| 1.5kU/kg | Intravenous Administration | 0.64 ± 0.11 | 1.32 ± 0.40 |
| 1.5kFU/kg | Intratracheal administration | 0.25 ± 0.06 | 43.5 ± 9.52 |
| 15.0kU/kg | Intratracheal administration | 1.15 ± 0.34 | >20 |
| 60kU/one time | Pulmonary inhalation administration | 0.04 ± 0.02 | 7.55 ± 0.99 |
| 300kU/one time | Pulmonary inhalation administration | 0.12 ± 0.04 | 17.3 ± 1.22 |

As seen from the results in the above Table, when the PC-SOD was intratracheally administered to bleomycin-induced lung injury model mice, the PC-SOD concentration in the lung tissue was higher about 33 times than that in the serum.
When the PC-SOD was administered through pulmonary inhalation, the PC-SOD concentration in the lung tissue was higher about 150 times than that in the serum.

### Example 2: Effect of intratracheal administration of PC-SOD for bleomycin-induced lung injury (acute) model mouse

To ICR mice (6- to 8-week old, body weight: 28 to 32 g), 5 mg/kg of bleomycin was intratracheally administered to produce lung injury. The confirmation of production of lung injury was performed in the same way as in Example 1.
A PC-SOD having a concentration of 0.15, 0.75, 1.5, 3.0, 15, or 30 kU/kg was dissolved in 5% xylitol, the PC-SOD was intratracheally administered to these lung injury model mice once daily for 3 days in a dose of 15 µL per mouse.
After 3 days, the alveolar lavage fluid was collected from the mice, and the whole cells were counted. Further, the respective cells in alveolar macrophages, lymphocytes, and neutrophils were stained by Diff-Quik stain, and the cells were counted.

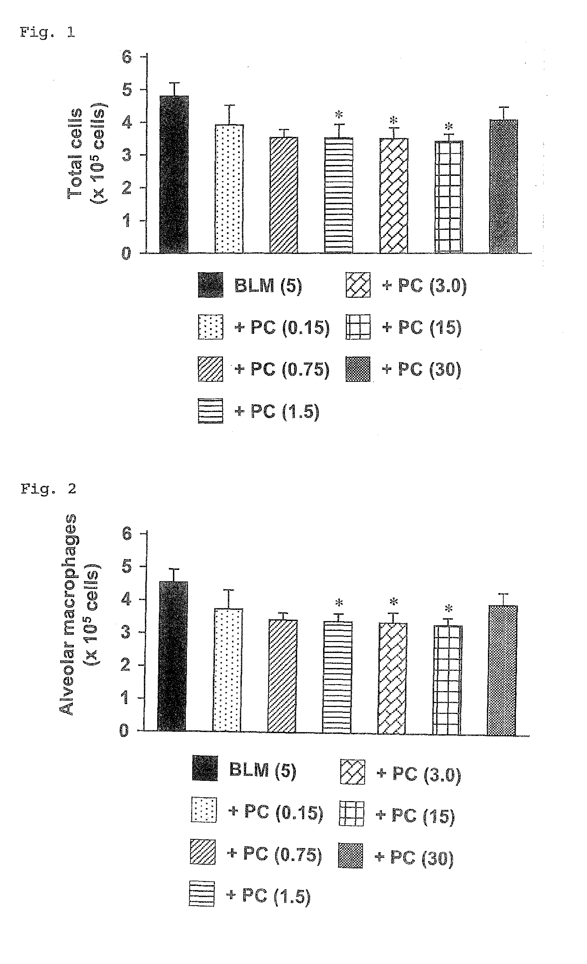

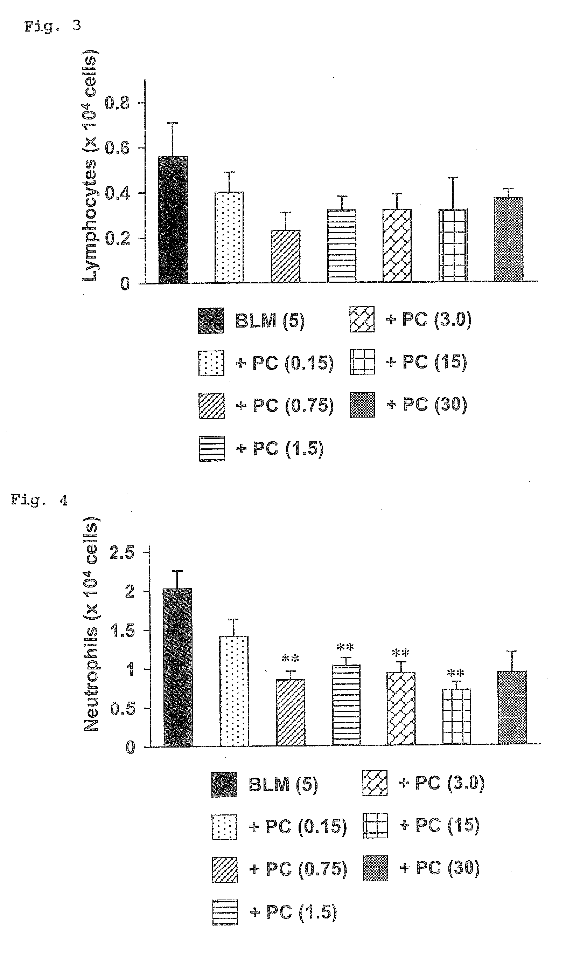

Fig. 1 shows the whole number of cells in the alveolar lavage fluid, Fig. 2 shows the results of alveolar macrophages, Fig. 3 shows the results of lymphocytes, and Fig. 4 shows the results of neutrophils.
In the drawings, * shows a significant difference to the bleomycin administration group.
As seen from the results shown in the Drawings, when the PC-SOD was intratracheally administered to the bleomycin-induced acute lung injury model mice, it was clear that the PC-SOD in the administration amount of 0.75 kU/kg or more significantly suppressed the injury.

### Example 3: Effect of intratracheal administration of PC-SOD for bleomycin-induced lung injury (chronic) model mouse

To ICR mice (6- to 8-week old, body weight: 28 to 32 g), 5 mg/kg of bleomycin was intratracheally administered to produce lung injury. The confirmation of production of lung injury was performed in the same way as in Example 1.
A PC-SOD having a concentration of 0.15, 0.75, 1.5, or 15 kU/kg was dissolved in 5% xylitol, and the PC-SOD was intratracheally administered to the lung injury model mice once daily for 14 days in a dose of 15 µL per mouse.
On 15th day, the mice were sacrificed, and slices of a lung tissue were produced. The slices were stained by H&E stain and Masson's trichrome stain, and change of the lung tissue was observed. In addition, the amount of hydroxyproline (collagen) in the lung tissue was determined, and the state of fibrosis in the lung was observed.
As a control, an example where 5% xylitol solution in which a PC-SOD was not dissolved was administered was used.

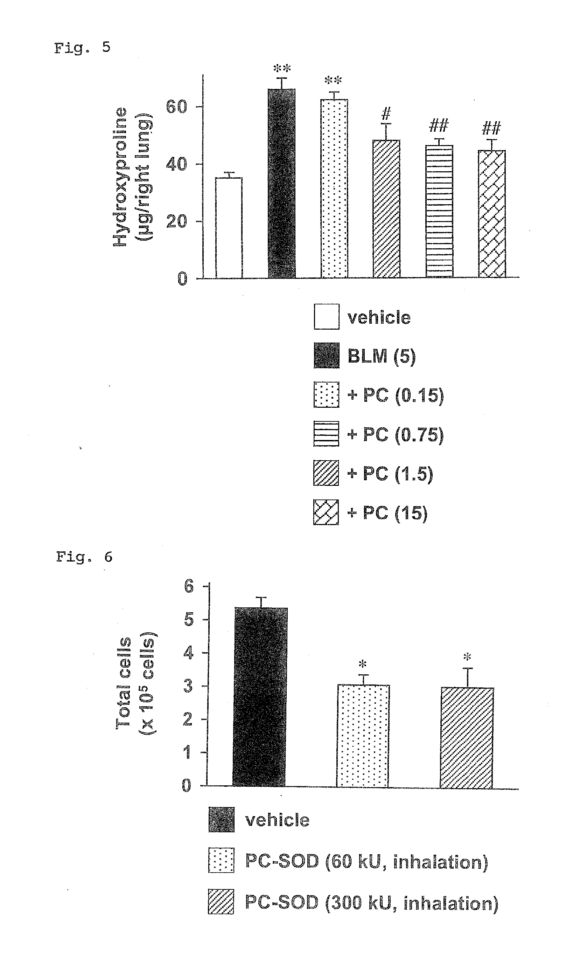

When the PC-SOD was intratracheally administered to bleomycin-induced chronic lung injury model mice, in the observation of the lung tissue, infiltration of the lung tissue was not found in the mice, in which 0.75 kU/kg or more of PC-SOD was administered.
The results of amount of hydroxyproline in the lung tissue are shown in Fig. 5.
In the drawings, * and # show a significant difference to the control and bleomycin administration groups, respectively.
As seen from the results shown in the drawings, when the PC-SOD was intratracheally administered to bleomycin-induced chronic lung injury model mice for inhalation to the lung, it was clear that the PC-SOD in the administration amount of 0.75 kU/kg or more significantly suppressed the injury.

### Example 4: Effect of administration of PC-SOD through pulmonary inhalation for bleomycin-induced lung injury (acute) model mouse

To ICR mice (6- to 8-week old, body weight: 28 to 32 g), 5 mg/kg of bleomycin was intratracheally administered to produce lung injury. The confirmation of production of the lung injury was performed in the same way as in Example 1.
For the lung injury model mice, a PC-SOD having a concentration of 60 or 300 kU was dissolved in 10 mL of solution, the PC-SOD was administered to the respective mice using an ultrasonic nebulizer through pulmonary inhalation once daily for 3 days.
After 3 days, the alveolar lavage fluid was collected from the mice, and the whole cells were counted. Further, the respective cells in alveolar macrophages, lymphocytes, and neutrophils were stained by Diff-Quik stain, and the cells were counted.

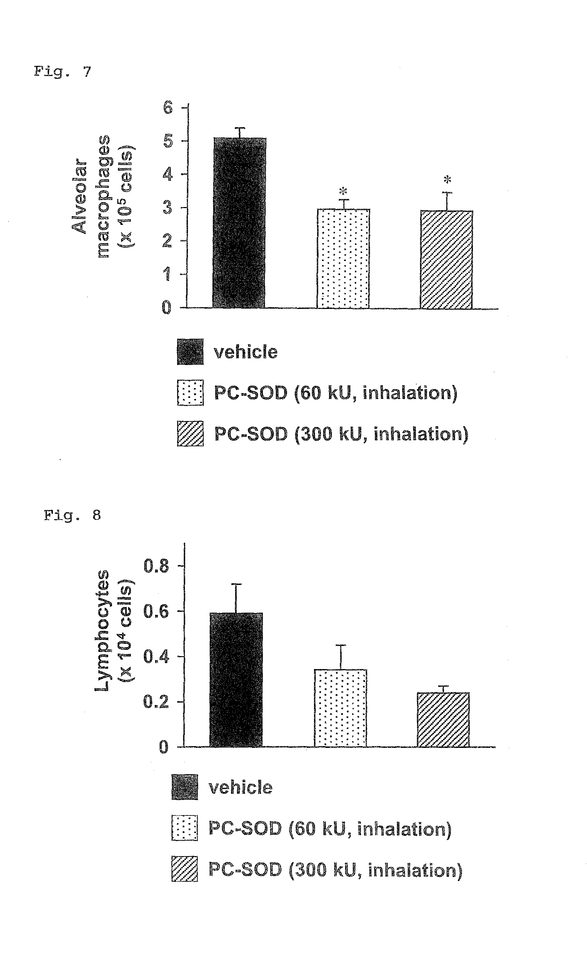

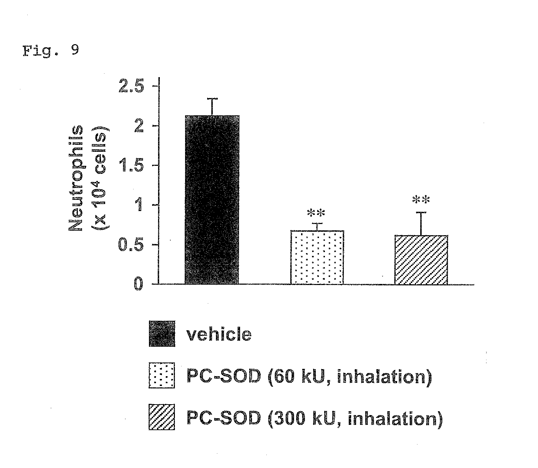

Fig. 6 shows the whole number of cells in the alveolar lavage fluid, Fig. 7 shows the results of alveolar macrophages, Fig. 8 shows the results of lymphocytes, and Fig. 9 shows the results of neutrophils.
In the drawings, * shows a significant difference to the bleomycin administration group.
As seen from the results shown in the drawings, when the PC-SOD was administered to bleomycin-induced acute lung injury model mice through pulmonary inhalation, it was clear that the PC-SOD in the administration amount of 60 kU significantly suppressed the injury.

From the results of Examples 1 to 4, a PC-SOD as the active ingredient of the present invention was administered to the bleomycin-induced lung injury model mice through intratracheal route or pulmonary inhalation (nebulizer) to significantly suppress the injury. Therefore, it is confirmed that the inhalant of the present invention is useful for therapeutic effect of lung diseases such as interstitial pneumonia.
Based on the results, clinical test results for human are shown in Examples described below.

### Example 5: Clinical test example of administration of PC-SOD by nebulizer

1. Investigation of safety of administration of PC-SOD by nebulizer to healthy male adults without liver or kidney dysfunction [Test Subjects] Eight healthy male adults aged 20 to 50 years, in which informed consent had been taken sufficiently before the test and agreement was obtained.

### [Processes] A placebo-controlled, single-blind study was performed.

### (1) Single-dose administration:

Eight healthy people were blindly divided into a PC-SOD administration group of 6 people and a placebo administration group of 2 people. 40 mg of PC-SOD and indistinguishable placebo, which had been dissolved in distilled water for injection, were administered once (single dose) by a nebulizer over about 30 minutes to the PC-SOD administration group and the placebo administration group, respectively.
Verification of safety was determined from abnormalities of clinical symptom and laboratory values by specialized physicians.
As a result, there were no differences in safety between the PC-SOD and placebo administration groups.

### (2) Repetitive dose:

Eight healthy people were blindly divided into a PC-SOD administration group of 6 people and a placebo administration group of 2 people. 16 mg of PC-SOD and indistinguishable placebo, which had been dissolved in distilled water for injection, were administered by a nebulizer over about 30 minutes to the PC-SOD administration group and the placebo administration group, respectively.
The administration was performed once daily in the morning. Continuous administration for 7 days was performed, and the clinical symptom in the hospital was observed.
Verification of safety was determined from abnormalities of clinical symptom and laboratory values by specialized physicians.
As a result, there were no differences in safety between the PC-SOD and placebo administration groups.

2. Investigation of effectiveness and safety of administration of PC-SOD by nebulizer to patients with moderate idiopathic interstitial pneumonia without liver or kidney dysfunction [Test Subjects] 8 patients with idiopathic interstitial pneumonia, aged 20 to 80 years, regardless of sex, in which informed consent had been taken well before the test and agreement was obtained.
The patients with idiopathic interstitial pneumonia were a patient who had been diagnosed with idiopathic interstitial pneumonia by X-ray and HRCT.

### [Processes]

Eight patients with idiopathic interstitial pneumonia were blindly divided into a PC-SOD administration group of 6 patients and a placebo administration group of 2 patients. 8 mg of PC-SOD and indistinguishable placebo, which had been dissolved in distilled water for injection, were administered by a nebulizer over about 30 minutes to the PC-SOD administration group and the placebo administration group, respectively.
The administration was performed once daily in the morning. Continuous administration for 28 days was performed, and the clinical symptom in the hospital was observed.
The effectiveness is evaluated by SP-A (note 1) which is a biomarker of pneumonia known as factors predicting the prognosis of idiopathic interstitial pneumonia.

### [Results]

In the PC-SOD administration group, an SP-A value after administration was significantly reduced, as compared with the value before the administration, and the SP-A value was improved as compared with the placebo administration group.
Safety was determined from abnormalities of clinical symptom and laboratory values by specialized physicians.
As a result, there were no differences in safety between the PC-SOD and placebo administration groups.
Note 1: Brent W. Kinder, Serum Surfactant Protein-A is a Strong Predictor of Early Mortality in Idiopathic Pulmonary Fibrosis. CHEST 2009; 135: 1557-1563

From the results of the above clinical test examples, the PC-SOD which is the active ingredient of the present invention is free of side effects, and thus safe for human. By administration through pulmonary inhalation (nebulizer) for lung diseases such as interstitial pneumonia, the PC-SOD is approved to be useful for therapeutic effects of interstitial pneumonia.

### Example 6: Formulation examples

### Liquid formulation for inhalation (1)

A liquid formulation for inhalation was prepared by dissolving 1%(w/w) of PC-SOD, 10%(w/w) of sucrose, and 0.05%(w/w) of benzalkonium chloride in an aqueous solution of 5% xylitol.

### Liquid formulation for inhalation (2)

A liquid formulation for inhalation was prepared using 1%(w/w) of PC-SOD, 10%(w/w) of sucrose, 0.05%(w/w) of benzalkonium chloride, 10%(w/w) of polyethylene glycol, 20%(w/w) of propylene glycol, and the balance of purified water.

### Powder formulation for inhalation

A powder formulation for inhalation was prepared using 5%(w/w) of PC-SOD and the balance of sucrose (fine powder).

### INDUSTRIAL APPLICABILITY

As described above, the inhalant provided by the present invention is an inhalant, in which a specific PC-SOD as an SOD is used as an active ingredient, has an excellent affinity for a cell membrane and the like as compared with the conventional SOD, and has a high ability to favorably eliminate superoxide anions locally in the lesion.
The present invention is an inhalant which allows the PS-SOD to be administered directly to a lung tissue or the like by inhalation. The effect of SOD eliminates active oxygens such as superoxide anions which induce cell injury to effectively suppress the induction, and as a result, treatment useful for interstitial pneumonia can be achieved, and has a great value in medical care.

## Claims

1. An inhalant comprising, as an active ingredient, a lecithinized superoxide dismutase represented by the following general formula (I):
SOD'(Q-B)ₘ (I)
(wherein SOD' is a residue of a superoxide dismutase; Q is a chemical crosslinking; B is a residue without a hydrogen atom of a hydroxyl group of lysolecithin having the hydroxyl group at a 2-position of glycerol; and m is an average number of bonds of lysolecithin to one molecule of superoxide dismutase and is an integer of 1 or more).

2. The inhalant according to claim 1, wherein Q in the formula (I) is -C-(O) -(CH₂)ₙ-C(O) - (wherein n is an integer of 2 or more).

3. The inhalant according to claim 1 or 2, wherein the SOD' is a residue of a human superoxide dismutase.

4. The inhalant according to claim 1 or 2, wherein the SOD' is a residue of a modified superoxide dismutase, in which an amino acid at a 111-position of amino acid sequence of a human superoxide dismutase is converted into S-(2-hydroxyethylthio)cysteine.

5. The inhalant according to claim 3 or 4, wherein the superoxide dismutase is a superoxide dismutase containing copper and zinc at an active center.

6. The inhalant according to any of claims 2 to 5, wherein n is an integer of 2 to 10.

7. The inhalant according to any of claims 1 to 6, wherein m is an integer of 1 to 12.

8. The inhalant according to any of claims 1 to 7, further comprising a stabilizing agent.

9. The inhalant according to claim 8, wherein the stabilizing agent is a sugar.

10. The inhalant according to claim 9, wherein the sugar is sucrose.

11. The inhalant according to claim 9, wherein the sucrose is sucrose processed by activated charcoal.

12. The inhalant according to claim 1, having a form of a fine powder formulation for inhalation.

13. The inhalant according to claim 1, having a form of an aqueous solution for inhalation or a suspension formulation for inhalation.

14. The inhalant according to any of claims 1 to 13, being intratracheally administered.

15. The inhalant according to any of claims 1 to 13, for use in treatment or prevention of interstitial pneumonia.

16. The inhalant according to any of claims 1 to 13, wherein interstitial pneumonia occurs as a side effect of antitumor drugs.
